# EUROPEAN PATENT APPLICATION

(11) **EP 1 598 043 A1**
(43) Date of publication of application: **23.11.2005**
(21) Application number: 03707145.3
(22) Date of filing: 27.02.2003
(51) Int. Cl.: A61G 12/00

(54) **SYRINGE NEEDLE REMOVING DEVICE FOR DENTAL CARTRIDGE TYPE SYRINGES**

(71) Applicant: Showa Yakuhin Kako Co., Ltd, Tokyo 104-0031 (JP); Hios Inc., Matsudo-shi, Chiba 270-2223 (JP)
(72) Inventor: TANAKA, Fumio, c/o SHOWA YAKUHIN KAKO CO., LTD., Tokyo 104-0031 (JP); ONO, Kazuhiro, c/o SHOWA YAKUHIN KAKO CO., LTD., Tokyo 104-0031 (JP); KITAGAWA, Ikuo, c/o HIOS INC., Matsudo-Shi, Chiba 270-2223 (JP); HAYASHI, Renji, c/o HIOS INC., Matsudo-Shi, Chiba 270-2223 (JP); KATO, Yoshinori, c/o HIOS INC., Matsudo-Shi, Chiba 270-2223 (JP); WAKABAYASHI, Kiyoshi, c/o HIOS INC., Matsudo-Shi, Chiba 270-2223 (JP)
(74) Representative: Alexander, Thomas Bruce
(86) International application number: PCT/JP2003/002260
(87) International publication number: WO 2004/075806

(57) **Abstract**

There is provided an injection needle removing apparatus for dental cartridge syringe comprising a chuck device (22) mounted within a housing (11) and including chuck jaws (26) for gripping a needle hub (25'), means for opening and closing the chuck jaws, a needle receptacle (14) adapted to rotatably support the housing (11), and means (50, 55, 56) for rotating the housing relative to the receptacle, the rotation of the housing causing the chuck device, together with the housing, to be rotated relative to the injection needle, thereby removing the injection needle from the syringe. In this arrangement, any rotative power transmission mechanism is not required to rotate the chuck device itself relative to the syringe.

## Description

### Field of the Invention

This invention relates to an apparatus for removing injection needles from dental cartridge syringes.

### Background Art

In dental treatment, injection of local anesthesia is carried out by means of a syringe for tooth extraction, surgical removal of the gums, incisions, marrow extraction, formation and the like. An anesthetic is usually commercially available in the form of a glass cartridge filled with the anesthetic solution. A rubber disc is fitted in the cartridge at its forward end and sealingly retained in place by a cap of aluminum having a centrally disposed aperture through which a portion of the rubber disc is exposed to the exterior. A plunger rubber is fitted in the cartridge at its rear open end and adapted to be pushed for ejection of the anesthetic solution contained in the cartridge. An injection needle is a double ended needle passing through and secured to a needle hub having female threads adapted to be threadedly engaged with male threads provided at the forward end of a barrel of the syringe. After loading the barrel with the cartridge, the needle hub is mounted on the barrel of the syringe. At this point, the rubber disc in the cartridge is penetrated with the butt end point to establish fluid communication of the injection needle with the anesthetic solution in the cartridge. Injection into the mouth cavity of a patient in procedure of local anesthesia is carried out by operating a plunger of the syringe to push the plunger rubber, injecting the anesthetic solution through the injection needle into the gum and further into a deep part.

Upon completion of the local anesthesia by the injection, removal of the injection needle from the barrel of the syringe is required for disposal of the injection needle. During operation of removal of the injection needle, there may be a danger of pricking the fingers of a dentist or co-staff with the used injection needle. Since a problem with virus infection to the dentist or co-staff due to such a needle pricking incident may be arise, there is a demand for developments of an apparatus capable of removing the used injection needle from the barrel without using the hands and fingers.

Such needle removing apparatuses have been proposed, for example, in Japanese Patent Publication Showa 63-216577, Japanese Patent Publication Heisei 7-241339, Japanese Patent Publication Heisei 8-80349, and Japanese Patent Publication Heisei 8-112320. Apart from Japanese Patent Publication Showa 63-216577, the needle removing apparatus disclosed in each of the other publications basically comprises a chuck device including chuck jaws for gripping the needle hub, means for actuating the chuck device and rotating it, and means for transmission of rotative power from a single drive source , for example, an electric motor to the actuating and rotating means. In this arrangement, the apparatus may be complicated and necessarily become a large size. This would make it difficult to provide an inexpensive apparatus. Japanese Patent Publication Showa 63-216577 appears to provide an apparatus which is relatively simple in construction but many matters of design have to be considered for practical use .

### Disclosure of the Invention

An object of the present invention is to provide an injection needle removing apparatus which is of simple construction and capable of rotating a chuck device without need for any rotative power transmission mechanism.

Another object of the invention is to provide such an injection needle removing apparatus wherein operation of a chuck device is performed by linear motion of a solenoid.

According to the invention, the above-mentioned objects can be achieved by providing an injection needle removing apparatus for dental cartridge syringe comprising a housing including a cover with an circular opening for insertion of an injection needle attached to a syringe and a support having a passage in alignment with the circular opening, a chuck device including chuck jaws mounted on the support of the housing and arranged to grip the needle hub from three ways, means for opening and closing the chuck jaws when the injection needle is inserted in the circular opening, a needle receptacle adapted to rotatably support the housing, and means for rotating the housing relative to the receptacle, the rotation of the housing causing the chuck device, together with the housing, to be rotated relative to the injection needle, thereby removing the injection needle from the syringe so that the removed injection needle can fall through the passage into the receptacle.

In a preferred embodiment of the invention, each of the chuck jaws is rotatably mounted on a support pin secured to the support of the housing, and the chuck device includes a turning support plate mounted on support pins through slots for turning the chuck jaws around the support pins to open and close the chuck jaws, and a solenoid device actuated to turn the turning support plate a predetermined angle.

In a further preferred embodiment of the invention, the means for rotating the housing relative to the receptacle includes a reduction geared motor fixed to the housing, a spur gear fixed to an output shaft of the motor, and a ring gear on the receptacle, the spur gear meshing with the ring gear to rotate the housing relative to the receptacle when the housing is connected to the receptacle.

### Brief Description of the Drawings

Figure 1 is a perspective view of an injection needle removing apparatus according to the invention and a syringe;
Figure 2 is a top perspective view of a portion of the injection needle removing apparatus according to the invention with a cover removed;
Figure 3 is a vertical cross-sectional view of the apparatus according to the invention;
Figure 4 is a top plan view of the apparatus showing chuck jaws in their opened position; and
Figure 5 is a view similar to Fig. 4, showing the chuck jaws in their closed position.

### Best Mode for Embodying the Invention

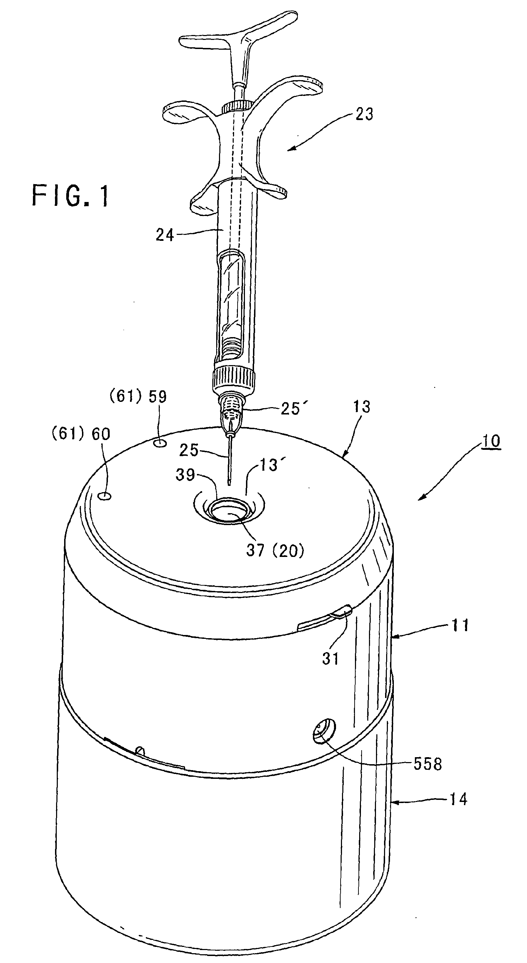

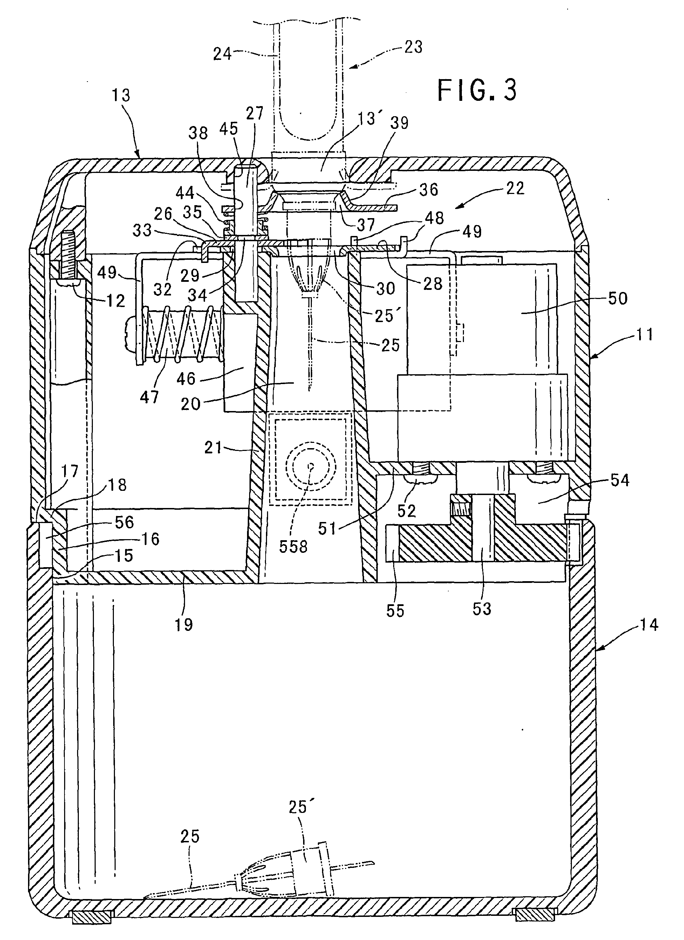

Referring to Figures 1 and 3 of the drawings, an injection needle removing apparatus according to the invention includes a cylindrical housing 11, and a cover 13 secured to the housing 11 by set-screws 12 only one of which is shown in Figure 3, and having a centrally disposed circular opening 13'. The housing 11 and the cover 13 is preferably of plastic moldings. The housing 11 has a reduced diameter portion 16 fitted in a circular opening 15 of a receptacle 14 and a shoulder 18 abutting the end face 17 of the receptacle 14, defining the circular opening 15. The housing 11 is provided with a hollow support 21 extending upwardly from a bottom wall 19 thereof in coaxial relation to the housing 11 and having a centrally disposed passage 20.

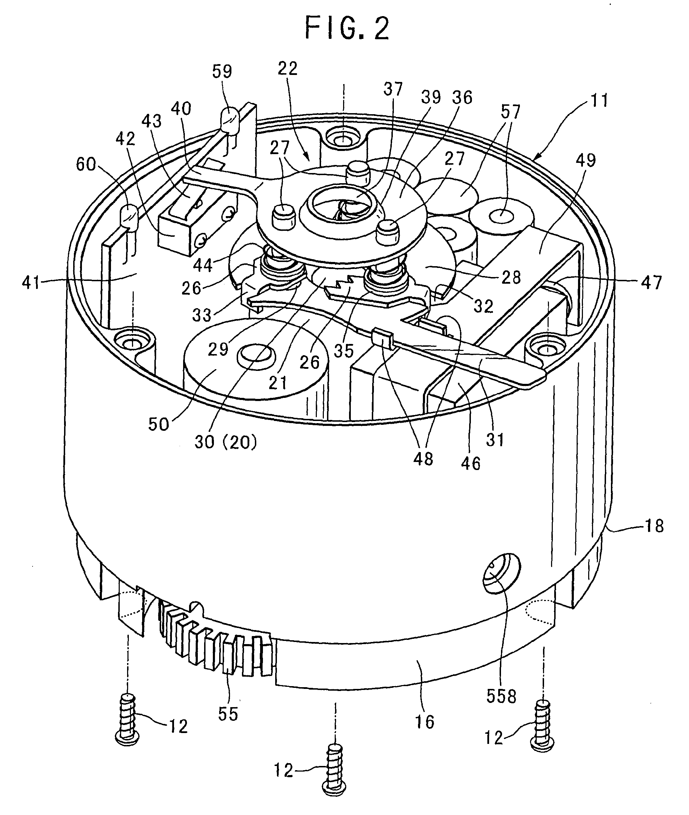

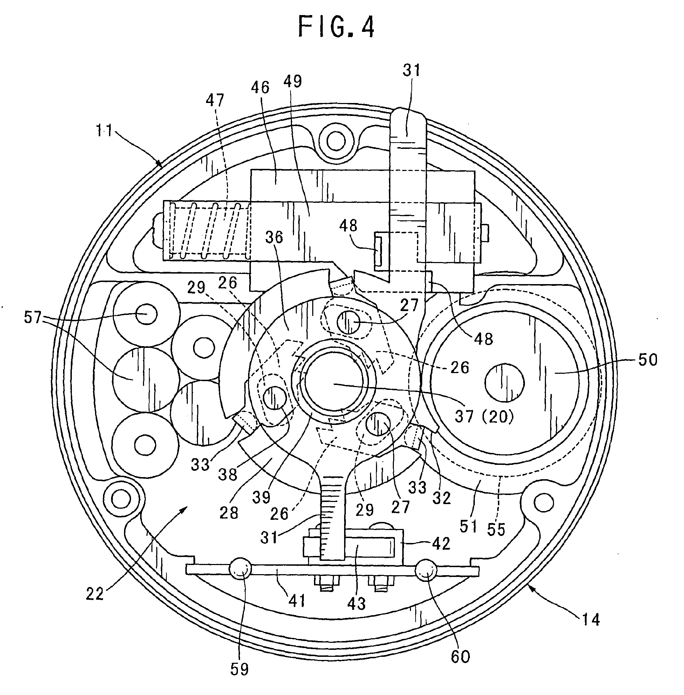

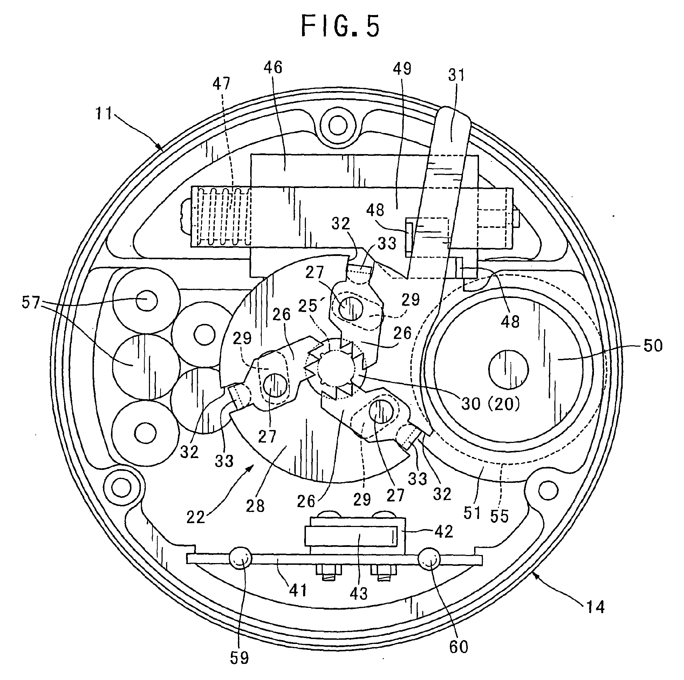

As shown in Figures 2, 4 and 5 of the drawings, a chuck device 22 is disposed at the top end of the hollow support 21 and includes 3- way chuck jaws 26 adapted to grip a needle hub 25' of an injection needle 25 charged in a barrel 24 of a syringe 23. Each of the chuck jaws 26 is rotatably mounted on one of three circumferentially spaced-apart support pins 27 fixed to the support 21 and extending upwardly from an upper surface of the support 21. Turning actuator plate 28 is interposed between the upper surface of the support 21 and the chuck jaws 26 and retained on the support 21 by the support pins 27 each received in one of slots 29 formed in the actuator plate 28 such that it can be turned a predetermined angle. The actuator plate 28 has a central hole 30 in alignment with the passage 20 in the hollow support 21 and an integral arm 31 for turning the actuator plate 28. In order that by turning the actuator plate 28, the chuck jaws 26 can be turned around the support pins 27, each of the chuck jaws 26 is operatively connected to the actuator plate by engaging a rearward bend 33 of each chuck jaw 26 with one of notches 32 formed in the actuator plate 28. The chuck jaws 26 may also be retained on the actuator plate 28 by fitting each of snap rings 34 in one of annular grooves 34 formed in the support pins 27.

A movable disk 36 has a circular opening 37 formed at the center thereof in alignment with the hole 30 in the actuator plate 28, and three apertures 38 formed in the movable disk around the central circular opening 37. The movable disk 36 is mounted on the support pins 27 for vertical movement by loosely passing the support pins 27 in the apertures 38. An annular rise 39 may be formed on the movable disk 36 around the central circular opening 37. The movable disk 36 has an radially outwardly extending arm 40 which is located above an operating lever 43 of a microswitch 42 on a printed circuit board 41mounted in the housing 11. A coil spring 44 is disposed around each of the support pins 27 between the movable disk 36 and each of the chuck jaws 26 to urge upwardly the movable disk 36, thereby preventing the arm 40 from depressing the switching lever 43 of the microswitch 42. As the movable disk 36 is pushed down against the action of the coil spring 44, the arm 40 of the movable disk 36 depresses the switching lever 43 to switch close the microswitch 42.

The cover 13 has recesses 45 formed on the lower surface thereof around the central circular opening 13' and receiving the tops of the support pins 27 so that the cover 13 can be rested on the housing 11 at a predetermined position. When the cover 13 is rested on the housing 11, the annular rise 39 is received in the circular opening 13' in the cover 13 so that it can be exposed to the exterior.

A solenoid 46 is fixedly secured inside the housing and provided with a plunger 47 which performs liner movement upon energizing the solenoid 46. The solenoid 46 also includes a reversed U-shaped bridge member 49 secured to the plunger 47 and having a pair of spaced-apart lugs 48. The arm 31 of the actuator plate 28 is located between the lugs 48 and 48 of the bridge member 49 so that the liner movement of the bridge member 49 causes the actuator plate 28 to be turned via the arm 31.

A reduction geared electric motor 50 is secured to a raised bottom portion 51 of a bottom wall 19 of the housing 11 by means of set-screws 52 and has a output shaft 53 extending downwardly through the raised bottom portion 51 by which a space 54 is defined. A spur gear 55 is fixed to the output shaft 53 within the space 54. A ring gear 56 is formed in the inner wall of the receptacle 14 adjacent to the circular opening 15, and when the reduced diameter portion 16 of the housing 11 is fitted in the circular opening 15 of the receptacle 14, the spur gear 55 meshes with the ring gear 56. Within the housing, there is provided a battery 57 used as a power source for the electric motor and solenoid. Reference numeral 58 indicates a jack in which a plug of a electric cord from a charger is inserted to charge the battery 57. When the plug is inserted in the jack 58, the electric motor is intended not to be energized even if the microswitch 42 is operated. Two luminous diodes 59, 60 on the printed circuit board 41 are located in two holes 61 in the cover 13, and one 59 of the luminous diodes indicates the operation of the apparatus while the other 60 indicates charging of the battery.

As can be seen in Figure 1, an injection needle 25 is attached to a syringe 23 by screwing a needle hub 25' onto threads on the barrel 24 at its forward end. In case that the injection needle 25 is to be unscrewed from the syringe 23 upon completion of the procedure of local anesthesia, the injection needle 25 on the barrel 24 is inserted through the circular opening 13' in the cover 13 into the passage in the support 21. At this point, a forward annular shoulder of the syringe 23 abuts the annular rise 39 around the central circular opening 37 in the movable disk 36. As downward force is applied to the syringe 23 to push down the movable disk 36 against the action of the coil springs 44, the arm 40 of the movable disk 36 depresses the switching lever 43 to bring the microswitch 42 into "on" , thereby establish the closed circuit between the electric power source and the solenoid 46 and electric motor50 via a control device (not shown) for energizing them.

As the solenoid 46 is energized, the bridge member 49 is linearly moved by the solenoid plunger to push the arm 31 through the lugs 48, 48 in the same direction of the bridge member 49, thereby turning clockwise the actuator plate 28 a predetermined angle. Turning of the actuator plate 28 causes its operatively associated chuck jaws 26 to be turned clockwise to their closed position around the support pins 27. Since the needle hub 25' of the injection needle 25 is in a position facing the chuck jaws when the syringe 23 is moved downwardly through the circular opening 13', the needle hub 25' is gripped from three ways by the closed chuck jaws 26.

The electric motor 50 is energized to rotate anti-clockwise the spur gear 55 fixed to the output shaft thereof so that the spur gear 55 revolves around the ring gear 56 on the receptacle 14. Thus, the housing 11 is rotated relative to the receptacle to rotate the chuck device 22 on the support integral with the housing, relative to the syringe. Since the syringe 23 is held by hand, the rotation of the chuck device 22 permits the needle hub 25' of the injection needle 25 to be rotated anti-clockwise relative to the cartridge holder 24 of the syringe 23, thereby unscrewing the needle hub 25' from the cartridge holder. The injection needle 25 remains gripped by the chuck jaws as long as the solenoid has been energized. After a given time has passed, the circuit from the electric power source is opened via the control device to de-energize the solenoid 46 and electric motor 50. As the solenoid 46 is de-energized, the bridge member 49 is returned to its original position so that the actuator plate 28 is turned anti-clockwise to turn anti-clockwise the chuck jaws 26 to its opened position around the support pins 27. Thus, the needle fitting 25' of the injection needle 25 is released from the chuck jaws 26 so that the injection needle 25 can fall through the passage 20 in the support 21 into the receptacle 14 for storage.

Since the apparatus according to the invention is arranged such that the chuck device is rotated together with the housing, any rotative power transmission mechanism is not required to rotate the chuck device itself directly, thereby making the construction of the apparatus simpler and providing an apparatus of light weight.

## Claims

1. An injection needle removing apparatus for dental cartridge syringe comprising a housing including a cover with an circular opening for insertion of an injection needle attached to a syringe and a support having a passage in alignment with the circular opening, a chuck device including chuck jaws mounted on the support of the housing and arranged to grip the needle hub from three ways, means for opening and closing the chuck jaws when the injection needle is inserted in the circular opening, a needle receptacle adapted to rotatably support the housing, and means for rotating the housing relative to the receptacle, the rotation of the housing causing the chuck device, together with the housing, to be rotated relative to the injection needle, thereby removing the injection needle from the syringe so that the removed injection needle can fall through the passage into the receptacle.

2. The injection needle removing apparatus for dental cartridge syringe according to claim 1 wherein each of the chuck jaws is rotatably mounted on a support pin secured to the support of the housing, and the chuck device includes a turning support plate mounted on support pins through slots for turning the chuck jaws around the support pins to open and close the chuck jaws, and a solenoid device actuated to turn the turning support plate a predetermined angle.

3. The injection needle removing apparatus for dental cartridge syringe according to claim 1 wherein the means for rotating the housing relative to the receptacle includes a reduction geared motor fixed to the housing, a spur gear fixed to an output shaft of the motor, and a ring gear on the receptacle, the spur gear meshing with the ring gear to rotate the housing relative to the receptacle when the housing is connected to the receptacle.
